# EUROPEAN PATENT APPLICATION

(11) **EP 3 418 744 A1**
(43) Date of publication of application: **26.12.2018**
(21) Application number: 17753243.9
(22) Date of filing: 15.02.2017
(51) Int. Cl.: G01N 33/574, C12Q 1/02, G01N 33/48, G01N 33/53, G01N 33/543, G01N 33/50, G01N 33/58

(54) **NON-CLINICAL TEST METHOD CHARACTERIZED BY QUANTITATIVE EVALUATION OF EXPERIMENTAL ANIMAL SPECIMEN**

(30) Priority: 19.02.2016 JP 2016030419
(71) Applicant: Konica Minolta, Inc., Tokyo 100-7015 (JP)
(72) Inventor: GOUDA, Hideki, Tokyo 100-7015 (JP); SHIRAISHI, Takeshi, Tokyo 100-7015 (JP); ISODA, Takeshi, Tokyo 100-7015 (JP); KOYAMA, Noboru, Tokyo 100-7015 (JP); OKADA, Hisatake, Tokyo 1007015 (JP)
(74) Representative: Gille Hrabal
(86) International application number: PCT/JP2017/005588
(87) International publication number: WO 2017/141987

(57) **Abstract**

An object of the present invention is to provide a non-clinical test method which allows for the quality control of experimental animals having a transplanted tumor site, such as tumor-bearing mice, or experimental animals having a lesion site other than a transplanted a tumor site, and which is characterized by including the step of identifying, using a specimen collected from such an experimental animal, the profile of a lesion site, for example, a transplanted tumor site, of the experimental animal, by a quantitative technique.

## Description

### Technological Field

The present invention relates to a non-clinical test method for evaluating the efficacy, side-effects and the like of a drug or a candidate substance thereof, which method is carried out using an experimental animal.

### Background

In cancer research, there are cases where model animals produced by transplanting human-derived cancer (tumor) cells or tissue into experimental animals, for example, tumor-bearing mice produced using mice as experimental animals, are used as experimental systems in which *in vivo* environment is reproduced.

Examples of known tumor-bearing mice include: cultured cancer cell-transplanted mice, produced by transplanting cultured cells derived from tumor cells collected from patients into mice, and allowing the transplanted cells to grow within the mice; and patient-derived tumor-transplanted mice, produced by transplanting tumor tissue or tumor cells collected from patients into mice, and allowing the transplanted tissue or cells to grow within the mice. The use of such tumor-bearing mice enables to investigate the efficacy or safety (toxicity) of a drug or a candidate substance thereof, while its research is still in a drug discovery phase or non-clinical trial phase, which is carried out before initiating a trial with human subjects. Further, since it is possible to produce mice bearing tissues or cells derived from patients having cancers with various characteristics, a comparative evaluation can be carried out to find out the profile of patients for whom a drug works more effectively.

Cultured cancer cell-transplanted mice have classically been recognized as established experimental animals, because they can be produced using cells being easily transplanted into mice, which cells are cultured and stabilized in test tubes. The mice transplanted with cloned cells inherit the clonal elements, and are thus suitable for tests in which the use of tumor-bearing mice with smaller individual differences is desired.

On the other hand, patient-derived tumor-transplanted mice are produced by directly transplanting tumor tissue (tumor site) or tumor cells collected from patients into mice. In particular, patient-derived tumor-transplanted mice produced by transplanting tumor tissue derived from patients (humans) into mice with acquired immune deficiency, and allowing the tissue to grow within the body of the mice for a certain period of time, are referred to as PDX (Patient-derived tumor xenograft) model mice.

Since cells cultured in test tubes are not used in the thus produced PDX model mice, it is thought that the use thereof allows for the evaluation of drug efficacy or safety, in a state where actual human pathological conditions are more highly reproduced as compared to using conventional tumor-bearing mice. In recent years, diagnosis and treatment methods (methods of obtaining indices for use in diagnosis or treatment) employing a non-clinical test method using PDX model mice have been actively developed. This is because, the importance of tests using tumor-bearing mice retaining the complexities of cancer cells, including the factors (genetic mutations and the like) causing the complexities, has been recognized, and further, it is considered desirable to be able to repeatedly reproduce such complexities. PDX models can be produced using not only mice, but using various types of experimental animals.

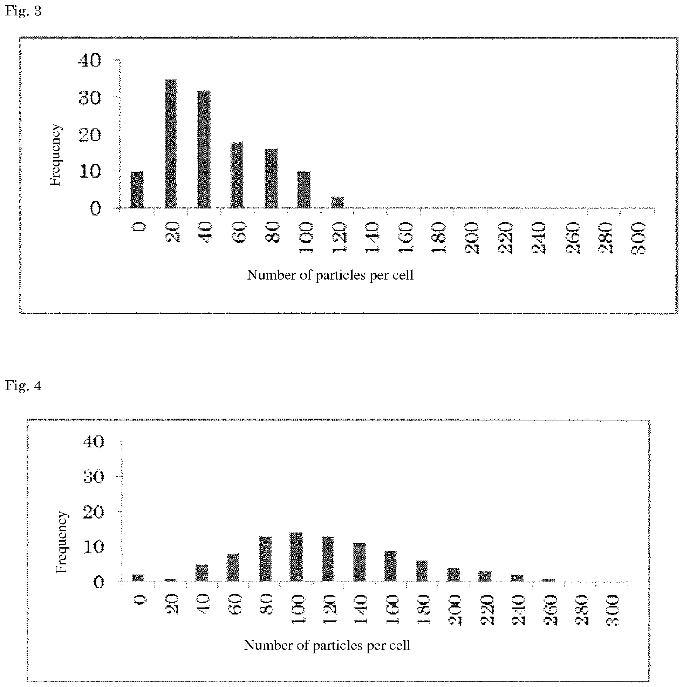

It has now been revealed that, even in PDX model experimental animals, in actual cases, the characteristics of cancer cells (expressed genes and expressed proteins) contained in tumor sites are not necessarily fully retained, and changes may occur as a result of passage. For example, Non-patent Document 1 describes the production of PDX model mice bearing bladder cancer. It is described therein that various conditions have been observed, for example, depending on individual patients or the type of proteins, in some of the mice, the characteristics of tumor tissue changed significantly from the specimens of original patients, and in some of the mice, such changes were not observed, or changes occurred gradually as a result of passage (see Figure 3, for example). In other words, although PDX model experimental animals inherit the genetic information of tumors through passage, but they are not as well cloned as cultured cancer cell-transplanted model animals, and accordingly, the characteristics of tumor cells in the first generation PDX model animals are not necessarily fully retained in passaged PDX model animals, and variations may occur among individuals even within the PDX model animals of the same strain.

In Non-patent Document 1, protein expression levels in the tumor tissues of PDX model mice were evaluated by an IHC (immunohistochemical) method. In the IHC method, an approach has been generally used in which an enzyme-labeled antibody is allowed to bind to a target protein (antigen) by a direct or indirect method, and then a substrate is allowed to react with the enzyme to develop color, such as, for example, DAB staining using a peroxidase and diaminobenzidine.

However, since the stain density depends greatly on the environmental conditions such as reaction temperature and reaction time, in the staining with an enzyme, such as the DAB staining in the IHC method, there is a problem that it is difficult to accurately estimate the actual amount of antigen and the like, from the stain density. Further, protein expression levels are often represented by several levels of scores based on the stain density and the like, as described in Non-patent Document 1, and thus, such an approach is more like a qualitative evaluation, rather than a quantitative evaluation.

At present, studies of academia are merely qualitatively analyzing protein expression levels in tumor tissues in patients and PDX mice by the IHC (immunohistochemical) method, as described in Non-patent Document 1. In addition, companies providing mice and those providing commissioned test services have little interest in accurately evaluating protein expression levels. It can be said that the technical significance of accurately and quantitatively determining the protein expression levels and the like in the tumor tissues, etc. of PDX mice, is yet to be known.

In recent years, methods for labeling proteins by nanosized fluorescent particles have been proposed, for example, particles obtained by integrating phosphors such as fluorescent dyes or quantum dots in a matrix such as a resin (Phosphor Integrated Dots: PIDs). and being utilizing.

At present, studies of academia are merely qualitatively analyzing protein expression levels in tumor tissues in patients and PDX mice by the IHC (immunohistochemical) method, as described in Non-patent Document 1. In addition, companies providing mice and those providing commissioned test services have little interest in accurately evaluating protein expression levels. It can be said that the technical significance of accurately and quantitatively determining the protein expression levels and the like in the tumor tissues, etc. of PDX mice, is yet to be known.

In recent years, methods for labeling proteins have been proposed, which utilize nanosized fluorescent particles, for example, particles (Phosphor Integrated Dots: PIDs) obtained by integrating phosphors such as fluorescent dyes or quantum dotsin a matrix such as a resin, and efforts are being made for the practical use thereof. By labeling a target protein using phosphor integrated dots, and irradiating an excitation light suitable for the fluorescent substance, the molecules of the protein can be observed as bright spots with high brightness. Accordingly, it is possible to quantitatively evaluate the amount of expressed protein, and to indicate the locations of the protein molecules, with a high accuracy. Further, observation and imaging can be performed for a relatively long period of time, since the fluorescence is less prone to discoloration. For example, WO 2012/029752 (Patent Document 1), WO 2013/035703 (Patent Document 2) and the like disclose methods in which immunostaining of target proteins is carried out using phosphor integrated dots (sometimes referred to as fluorescent substance-integrated nanoparticles).

### Related Art Documents

### Patent Documents

Patent Document 1: WO 2012/029752
Patent Document 2: WO 2013/035703

### Non-patent Document

Non-patent Document 1: Jager et al., Patient-derived bladder cancer xenografts in the preclinical development of novel target therapies. Oncotarget, Vol.6, No. 25, 21522-21532

### Summary

### Problems to be Solved by the Invention

Since the characteristics of tumor cells are not fully retained in patient-derived tumor-transplanted animals, such as PDX model mice, as described above, the characteristics of the tumor cells vary even among the model animals of the same strain, and it cannot be said that the quality control of these animals is sufficiently carried out in the current status. In cases where such PDX mice are used in the tests of drug efficacy and the like, it is ambiguous whether or not the outcomes which may occur in donor patients are reproduced in the mice, and the possibility remains that the analysis of the test results may not be reliable. To avoid such ambiguity, it is necessary, in general, to make a test plan using about five PDX mice as one group. This is considered to be one of the reasons making the use of PDX mice not as popular as it has been expected at first.

The present invention has been done in view of the above described problems, and an object of the present invention is to provide means for more accurately carrying out the quality control of experimental animals having a transplanted tumor site, such as PDX mice, and the analysis of the results of the tests using such experimental animals. Note that such means are not limited to solving the problems associated with tumor-bearing mice, including PDX mice, and can be used for solving the same problems associated with other lesion model animals (particularly, mice), such as, for example, Alzheimer's disease model mice, diabetes model mice, genetic disease model mice, and infectious disease model mice.

### Means for Solving the Problems

The present inventors have discovered that it is possible to more accurately carry out the above described quality control and the analysis of the test results: by identifying, using a specimen collected from an experimental animal, the profile of a transplanted tumor site of the experimental animal by a quantitative technique, preferably by immunostaining using fluorescent nanoparticles such as phosphor integrated dots; and by utilizing information obtained, for example, from the average expression level per cell of a target protein, a histogram showing the expression level per cell of the protein and the number of cells (frequency) corresponding thereto, and the like. Further, the present inventors have discovered that the non-clinical test method as described above for a tumor-bearing animal (mouse) can be applied to other model animals as described above.

In other words, the present invention provides, in one aspect, a non-clinical test method including the step of identifying, using a specimen collected from an experimental animal (lesion model animal), the profile of a lesion site, for example, a transplanted tumor site, of the experimental animal by a quantitative technique.

### Effect of the Invention

The present invention enables to carry out the quality control of experimental animals, for example, various types of model mice such as PDX mice, and the analysis of the results of the tests using such experimental animals, with a high level of accuracy. According to the present invention, it is expected that non-clinical tests using experimental animals will be more actively utilized hitherto unprecedented in various settings.

### Brief Description of the Drawings

FIG. 1 is a histogram showing the number of particles per cell and the frequency (number of cells) measured for the tumor sites of first generation mice (PI) which have been transplanted with the breast cancer tissue of a patient, produced in Example 3.
FIG. 2 is a histogram showing the number of particles per cell and the frequency (number of cells) measured for the tumor sites of second generation mice 1 (P2-1) produced in Example 3.
FIG. 3 is a histogram showing the number of particles per cell and the frequency (number of cells) measured for the tumor sites of second generation mice 2 (P2-2) produced in Example 3.
FIG. 4 is a histogram showing the number of particles per cell and the frequency (number of cells) measured for the tumor sites of third generation mice 1 (P3-1) produced from the P2-1 mice, in Example 3.
FIG. 5 is a histogram showing the number of particles per cell and the frequency (number of cells) measured for the tumor sites of third generation mice 2 (P3-2) produced from the P2-2 mice, in Example 3.

### Detailed Description of Embodiments

The non-clinical test method according to the present invention includes the step of identifying, using a specimen collected from an experimental animal, the profile of a lesion site of the experimental animal by a quantitative technique (sometimes referred to as "a lesion site profile identification step", in the present specification).

In cases where the experimental animal is a tumor-bearing animal model, such as a PDX model mouse, for example, the lesion site is a transplanted tumor site. In this case, the above described "a lesion site profile identification step" can be referred to as "a post-transplant profile identification step".

The "tumor site" refers to a site including "human-derived tumor tissue or tumor cells" and the "specimen" is collected from the tumor site. The tumor site usually contains, along with the human-derived tumor tissue or tumor cells, "substances other than human-derived tumor tissue".

In general, the "specimen collected from an experimental animal" is in the form of a sample slide prepared in accordance with a predetermined procedure, as commonly used in the case of evaluating the expression level of a target protein by immunostaining, or the like, for example, a sample slide conventionally used in the diagnosis of pathology, for evaluating the expression level of a target protein by immunostaining, etc. The non-clinical test method according to the present invention is carried out using such a specimenoutside the living body of the experimental animal.

The "profile" as used in the present invention refers to characteristics gathered from information such as, the expression level of a target protein, as well as the type, number and morphology of the cells expressing the target protein, and the expression sites of the protein (in cases where a tumor-bearing animal model is used as the experimental animal, the distribution within the tumor tissue or tumor site, and the occupied area).

The target protein to be identified in the profile of the present invention is not particularly limited, as long as it is expressed by the cells contained in the specimen. However, the target protein is preferably one which is abundantly expressed in these cells. One specific type of protein may be taken as the target protein, or two or more types of proteins may be taken as the target proteins.

In an example of a preferred embodiment of the present invention, the target protein could be a marker targeted of an already developed molecular target drug, and the profile to be identified includes the expression level of the marker, and the expression distribution thereof within the tumor tissue or tumor site. Various types of such markers are known, and examples thereof include HER2, HER3, PD-L1, PD-1, CTLA-4, EGFR, and VEGFR.

In an example of a preferred embodiment of the present invention, the target protein is a phosphorylated protein, and the profile to be identified includes the expression level of the phosphorylated protein, and the expression distribution thereof within the tumor tissue or tumor site. Examples of such a phosphorylated protein include HER2, HER3, EGFR, and VEGFR. The phosphorylated protein can be quantified, by using an antibody which specifically recognizes only the phosphorylated form from the target protein, when carrying out immunostaining. In cases where it is intended to quantify the target protein as a whole, regardless of the phosphorylated form or non-phosphorylated form, an antibody may be used which recognizes the target protein without discriminating these forms.

In cases where the specimen is a tumor tissue or tumor site, the tumor tissue or tumor site contains not only tumor cells, but also cells other than the tumor cells, such as, for example, immune cells which interact with the tumor cells. Accordingly, the definition of the target protein in the present specification preferably includes cancer-associated proteins in tumor cells and/or proteins in immune cells.

### (Cancer-associated Proteins in Tumor Cells)

Representative examples of the "cancer-associated proteins" include "immune-related proteins in cancer cells", "pathway-related proteins in cancer cells", and "metastasis-related proteins in cancer cells". Various types of cancer-associated proteins are known for each group of the proteins categorized as described above, and it is possible to select as an appropriate cancer-associated protein depending on the purpose of the diagnosis or treatment, without particular limitation. Cancer-associated gene expression panels provided by nCounter: an immune-related gene panel (Immune), a pathway-related gene panel (Pathway), and a metastasis-related gene panel (Progression), each covers 770 genes, and the proteins coded by the genes of these three panels correspond to the immune-related proteins, the pathway-related proteins, and the metastasis-related proteins in cancer cells, respectively. Further, mutant proteins corresponding to the mutant genes of these genes can also be included in the immune-related proteins, the pathway-related proteins and the metastasis-related proteins.

Examples of the "immune-related proteins in cancer cells" include immune checkpoint proteins, such as CD40, TL1A, GITR-L, 4-188-L, CX4D-L, CD70, HHLA2, ICOS-L, CD85, CD86, CD80, MHC-II, PDL1, PDL2, VISTA, BTNL2, B7-H3, B7-H4, CD48, HVEM, CD40L, TNFRSF25, GITR, 4-188, OX40, CD27, TMIGD2, ICOS, CD28, TCR, LAG3, CTLA4, PD1, CD244, TIM3, BTLA, CD160, and LIGHT.

Examples of the "pathway-related proteins in cancer cells" include: cancer cell growth factors and cancer cell growth factor receptors, such as EGFR (HER1), HER2, HER3, HER4, IGFR, and HGFR; cell surface antigens, vascular growth factors and vascular growth factor receptors, such as VEGF-A, VEGF-B, VEGF-C, VEGF-D, VEGF-E, P1GF-1, and P1GF-2; and cytokines and cytokine receptors, such as interferons, interleukins, G-CSF, M-CSF, EPO, SCF, EGF, FGF, IGF, NGF, PDGF, and TGF.

Examples of the "metastasis-related proteins in cancer cells" include: cancer progression markers such as ACTG2, ALDOA, APC, BRMS1, CADM1, CAMK2A, CAMK2B, CAMK2D, CCL5, CD82, CDKN1A, CDKN2A, CHD4, CNN1, CST7, CTSL, CXCR2, YBB, DCC, DENR, DLC1, EGLN2, EGLN3, EIF4E2, EIF4EBP1, ENO1, ENO2, ENO3, ETV4, FGFR4, GSN, HK2, HK3, HKDC1, HLA-DPB1, HUNKIL11, KDM1A, KISS1, LDHA, LIFR, MED23, MET, MGAT5, MAP2K4, MT3, MTA1, MTBP, MTOR, MYCL, MYH11, NDRG1, NF2, NFKB1, NME1, NME4, NOS2, NR4A3, PDK1, PEBP4, PFKFB1, PFKFB4, PGK1, PLAUR, PTTG1, RB1, RORB, SET, SLC2A1, SNRPF, SSTR2, TCEB1, TCEB2, TCF20, TF, TLR4, TNFSF10, TP53, TSHR, MMP, MMP2, MMP10, and HIF1.

### (Proteins in Immune Cells)

Examples of the "proteins in immune cells" include PD-1, CTLA-4, TIM3, Foxp3, CD3, CD4, CD8, CD25, CD27, CD28, CD70, CD40, CD40L, CD80, CD86, CD160, CD57, CD226, CD112, CD155, OX40 (CD134), OX40L (CD252), ICOS (CD278), ICOSL (CD275), 4-1BB (CD137), 4-1BBL (CD137L), 2B4 (CD244), GITR (CD357), B7-H3 (CD276), LAG-3 (CD223), BTLA (CD272), HVEM (CD270), GITRL, Galectin-9, B7-H4, B7-H5, PD-L2, KLRG-1, E-Cadherin, N-Cadherin, R-Cadherin, IDO, TDO, CSF-1R, HDAC, CXCR4, FLT-3, and TIGIT.

### (Proteins Contained in Stromal Tissue)

Further, the target protein in the present specification may be a protein which is expressed in cells other than tumor cells and immune cells. Specific examples of the protein which is expressed in cells other than tumor cells and immune cells include proteins contained in stromal tissue.

The "stromal tissue" is mainly composed of: stromal cells such as fibroblasts, endothelial cells, leukocytes (lymphocytes, monocytes, neutrophils, eosinophils, and basophils); and an extracellular matrix composed of proteins such as collagen and proteoglycan. Either of the amount of stromal cells or the amount of extracellular matrix may be measured, but it is preferred to measure the amount of stromal cells, which are considered to have a greater influence on the characteristics of the human-derived tumor cells carried by an experimental animal, and it is more preferred to measure the amount of protein expressed in fibroblasts, which are representative stromal cells.

For example, in cases where the experimental animal is a tumor-bearing animal model, such as a PDX model mouse, the "stromal tissue" contained in the tumor site is typically "stromal tissue derived from the experimental animal" or "stromal tissue of the human-derived tumor tissue". The stromal tissue derived from the experimental animal is a tissue which had mixed into the stromal tissue of the human-derived tumor tissue, during the period in which the tumor site was retained within the living body of the experimental animal. On the other hand, the stromal tissue of the human-derived tumor tissue is derived from a tissue included in the tumor tissue which had been collected from a human (patient) and transplanted into the experimental animal. The higher the ratio of the amount of the stromal tissue derived from the experimental animal with respect to the amount of the tumor tissue or the specimen, the relatively smaller the ratio of the amount of the stromal tissue of the human-derived tumor tissue.

As the protein contained in the stromal cells, it is possible to use, for example, any appropriate protein selected from the following membrane proteins, which are stromal cell markers. In particular, CD140a is a membrane protein which is expressed on the surface of cells such as fibroblasts, megakaryocytes, monocytes, erythrocytes, myeloid progenitor cells and endothelial cells, and is preferred as the stromal cell marker in the present invention.
CD106 (VCAM-1, INCAM-110; CD49d/ CD29-L) ... activated vascular endothelial cells, DC
CD109 (Platelet activation factor, 8A3, E123) ...activated T cells, platelets, vascular endothelium, megakaryocytes, CD34+ progenitor cell subsets
CD140a (PDGF-R, PDGFR2) ...fibroblasts, megakaryocytes, monocytes, erythrocytes, myeloid progenitor cells, endothelial cells
CD140b (PDGF-R, PDGFR1) ...endothelial cells, stromal cells
CD141 (Thrombomodulin) ...vascular endothelium, myeloid cells, platelets, smooth muscles
CD142 (Tissue Factor (TF), Thromboplastin) ...epithelial cells, activated monocytes, activated vascular endothelium
CD143 (ACE; angiotensin convertase) ...vascular endothelium, epithelial cells, activated macrophages
CD144 (VE-Cadherin, Cadherin-5: involved in vascular endothelial permeability) ...vascular endothelium
CD145 (7E9, P7A5) ...endothelial cells
CD146 (MUC18, s-endo, Mel-CAM) ...vascular endothelium, activated T cells, melanoma
CD147 (Basigin, M6, EMMRRIN) ...leukocytes, erythrocytes, vascular endothelium, platelets
CD201 (EPCR: Protein C receptor) ...vascular endothelium
CD202 (TIE2, TEK; Angiopoietin-1-R) ...vascular endothelium, hematopoietic stem cell subsets
CD280 (Endo180, TEM22, uPARAP (uPAR-associated protein); bone marrow progenitor cells, fibroblast, endothelial cell subsets, macrophage subsets
CD299 (DC-SIGN-related, L-SIGN (Liver/Lympho node specific ICAM3-grabbing nonintegrin)) ...endothelial cells
CD309 (VEGFR2: Vascular endothelial growth factor receptor 2, KDR) ...endothelial cells, megakaryocytes, platelets, stem cell subsets
CD322 (JAM2: Junctional adhesion molecule 2) ...endothelial cells, monocytes, B cells, T cell subsets
CD331 (FGFR1: Fibroblast growth factor receptor 1) ...fibroblasts, epithelial cells
CD332 (FGFR2, Keratinocyte growth factor receptor) ...epithelial cells
CD333 (FGFR3, JTK4; Achondroplasia, Thanatophoric dwarfism) ...fibroblasts, epithelial cells
CD334 (FGFR4, JTK2, TKF) ...fibroblasts, epithelial cells
CD339 (Jagged-1, JAG1; Alagille syndrome) ...stromal cells, epithelial cells.

### (Experimental Animal)

As the "experimental animal" in the present invention, any of various types of experimental animals, particularly, a lesion model animal can be used, depending on the object of the non-clinical test method. The experimental animal may be, for example, a tumor-bearing animal which already retains a tumor site derived from tumor cells or tumor tissue and grown *in vivo.* In addition, it is possible to use any of various types of lesion model animals, such as Alzheimer's model mice, diabetes model mice, genetic disease model mice, infectious disease model mice and the like, as the subject animal of the present invention. Examples of animal species include those which are genetically controlled to a certain extent and which satisfy the requirement of having homogeneous genetic traits, such as, for example, mice, rats, rabbits, guinea pigs, Mongolian gerbils, hamsters, ferrets, dogs, mini pigs, monkeys, cows, horses, and sheep.

In cases where a tumor-bearing animal model is used as the experimental animal, the technique for allowing the experimental animal to retain the tumor site is not particularly limited, and a known technique can be used.

For example, tumor-bearing model mice can be largely categorized into three types: naturally induced tumor-bearing mice, cultured cancer cell-transplanted mice, and patient-derived tumor-transplanted mice (see the following table; Kohrt et al., Defining the optimal murine models to investigate immune checkpoint blockers and their combination with other immunotherapies. Annals of Oncology 00: 1-9, 2016).

The cultured cancer cell-transplanted mice are produced by transplanting cultured cells derived from tumor cells collected from patients. Examples of mice transplanted with human-derived cultured cancer cells include CDX (Cell-line derived xenograft) model mice; and examples of mice transplanted with tumor tissue derived from humans (patients) include PDX (Patient derived xenograft) model mice, Immuno-avatar model mice, Hemato-lymphoid humanized model mice, and Immune-PDX model mice.

**[Table 1]**

| | Cancer cells | Immune cells | Model |
|---|---|---|---|
| Naturally induced tumor-bearing mice | murine | murine | Classic model produced by transplanting a carcinogen compound |
| | | | *Genetic-engineered mouse model |
| | | | *Human KI mice |
| Cultured cancer cell-transplanted mice | murine | murine | (3) Syngeneic murine model |
| | human | murine | (4) Cell-line derived xenograft (CDX) |
| Patient-derived tumor tissue-transplanted mice | human | murine | (5) Patient derived xenograft (PDX) |
| | | | (6) Immuno-avatar mice |
| | | | (7) Hemato-lymphoid humanized mice |
| | | | (8) Immune-PDX |

| | | | |
|---|---|---|---|
| *gene knock-in mice | | | |

The PDX mice are produced, as described above, by transplanting tumor tissues derived from patients into mice with acquired immune deficiency. Further, the Immuno-avatar model mice, the Hemato-lymphoid humanized model mice, and the Immune-PDX model mice are produced by transplanting tumor tissues derived from patients into mice with acquired immune deficiency which has been transplanted with human peripheral blood mononuclear cells, CD34+ human hematopoietic stem cells and the progenitor cells thereof (HSPC), or tumor-infiltrating lymphocytes, respectively.

The definition of the patient-derived tumor-transplanted mouse encompasses all of the following: a mouse which has been grown for a certain period of time after being transplanted with tumor tissue derived from a patient (0th generation); a first generation mouse into which the tumor site of the 0th generation mouse has been transplanted (passaged); and an nth+1 generation mouse into which the tumor site of a mouse of the nth generation (n ≥ 1) and beyond has been transplanted (passaged).

Accordingly, the expression "transplanted tumor site" in the present invention encompasses any of the tumor sites of experimental animals of from the 0th generation, transplanted with the tumor cells or tumor tissue of a patient or with cultured cells, to the nth+1 generation.

To produce a patient-derived tumor-transplanted animal, various types of techniques have been attempted, such as, for example: a technique in which a block of tumor is transplanted into an animal by cutting open the body thereof; a technique in which tumor tissue introduced into a frozen needle is injected into an animal by injection; a technique in which a matrix is introduced simultaneously upon transplantation of tumor tissue; and a technique in which a hormone such as estrogen is administered to an animal before the transplantation. In particular, the site to which the tumor is transplanted is preferably selected to suit the individual application, because the condition of the resulting tumor-bearing animal and the condition of the tumor vary greatly depending on the transplantation site. The type of transplantation can be largely classified into subcutaneous transplantation and orthotopic transplantation (a tumor generated in a certain organ is transplanted into the same organ of a donor animal), depending on the site of transplantation.

The subcutaneous transplantation is usually the first choice, because it is possible to subject the transplanted animal to a test after confirming that the transplanted tumor has grown to a certain size, since the gradual growth of the tumor can be confirmed by visual observation, and in addition, the transplantation process can be carried out easily. However, since the environment of the transplanted site (subcutaneous site) greatly differs from that of the site where the tumor originated, there are risks that a capsule may be formed around the tumor, the formation of blood vessels may be too slow to result in an insufficient tumor growth, and the like.

In the orthotopic transplantation, on the other hand, the formation of a capsule is less likely to occur, and in cases where tumor tissue derived from a human patient is transplanted, in particular, it has been reported that the transplanted tumor grows, reflecting the characteristics of the tumor of the patient. For example, it has been reported that, in cases where tissue derived from a patient with advanced lymph node metastasis is orthotopically transplanted into a mouse, lymph node metastasis progresses as the tumor grows (Proc. Natl. Acad. Sci. USA Vol. 89, pp. 5645-5649, June 1992).

### (Profile Information)

Of the information included in the profile identified in the non-clinical test method according to the present invention, examples of information regarding the expression level of the target protein and the number of cells expressing the protein include information regarding: (i) the average expression level per cell of the target protein; (ii) the expression level per unit area of the tissue, of the target protein; (iii) a histogram showing the expression level per cell of the target protein and the number of cells corresponding thereto; and (iv) a curve showing the expression level per cell of the target protein and the number of cells corresponding thereto, in a specimen (sample slide). The profile may include any one piece of such information alone, or a combination of a plurality of pieces of information. Further, a plurality of target proteins may be selected and a combination of the respective pieces of information thereof may be included in the profile.

In the case of quantifying (i) the average expression level per cell of the target protein, for example, a specimen (sample slide) is immunostained with fluorescent nanoparticles, and at the same time, also stained with a staining agent for morphological observation (such as eosin) so that the shape of the cells can be identified. The observation and imaging of the specimen in a dark field are carried out while irradiating an excitation light having a predetermined wavelength corresponding to the fluorescent nanoparticles, to obtain an image in which the fluorescent nanoparticles labeling the target protein are shown as bright spots. Meanwhile, the observation and imaging in a bright field are carried out, to obtain an image in which the shape of the cells are indicated by the staining. When the thus obtained two images are overlaid by image processing, it is possible to count the number of bright spots indicating the molecules of the expressed target protein, for each of the cells included in the entire image, or included in a specific area (for example, tumor tissue alone) in the image. The number of bright spots may be used as an index of the expression level of the target protein. Further, there is a case in which a plurality of fluorescent nanoparticles constitute one single bright spot, and in such a case, the number of fluorescent nanoparticles included in the one single bright spot can be calculated by dividing the brightness (brightness, fluorescence intensity) thereof by the brightness per one fluorescent nanoparticle separately measured in advance. The thus obtained number of the particles may be used as an index value of the expression level of the target protein. By determining the number of bright spots or the number of particles for all the cells included in the image, it is possible to quantify the average expression level per cell.

In the case of determining (ii) the expression level per unit area of the tissue, of the target protein, it can be achieved by: obtaining the total sum of the number of bright spots or the number of particles determined in the same manner as in (i), in the cells contained in the tissue present in a specific area in the image; and then dividing the sum by the area of the tissue.

In the case of preparing a (iii) a histogram showing the expression level per cell of the target protein and the number of cells corresponding thereto, first, the number of bright spots or the number of particles indicating the molecules of the expressed target protein is obtained, for each of the cells included in the entire image, or included in a specific area (for example, tumor tissue alone) of the image, in the same manner as in (i). Subsequently, the expression level per cell of the target protein is divided into sections every predetermined number of particles (for example, as carried out in the Examples in the present specification, the number of particles per cell of from one to 300 is divided every 20 particles, into 16 sections, including the section of 0) and plotted on the horizontal axis, and the number of cells (frequency) corresponding to each section is counted and plotted on the vertical axis, thereby preparing the histogram.

In the case of preparing (iv) a curve showing the expression level per cell of the target protein and the number of cells corresponding thereto, first, the number of bright spots or the number of particles indicating the molecules of the expressed target protein is obtained, for each of the cells included in the entire image, or included in a specific area (for example, tumor tissue alone) of the image, in the same manner as in (i). Subsequently, the expression level per cell of the target protein is plotted on the horizontal axis, continuously (without dividing into sections as in the case of preparing the histogram), and the number of cells (frequency) corresponding to each expression level is counted and plotted on the vertical axis, thereby preparing the curve.

From the histogram described in (iii) and the curve described in (iv), it is possible to obtain information regarding, for example, the state of the distribution (the shape of the histogram or the curve, the number of the peaks); the levels of values of the mean value or median value and the variance (CV); and in the case of the histogram, in particular, the level of the number of cells (frequency) corresponding to the section with the highest number of bright spots or particles per cell, and the like. By comparing such information with the test results of drug efficacy, stability or the like, it is possible to analyze and to understand, for example, to which piece of information the drug efficacy, the stability, or the like is most highly related, in other words, which piece of information is most adequate to be used for making a prediction of the drug efficacy, stability, or the like.

Further, the profile may include information other than the information regarding the cells expressing the target protein, such as for example, the vascular occupancy in the specimen (for example, tumor tissue or tumor site).

The expression "to identify by a quantitative technique" refers to identifying the profile as described above, particularly, the profile including the protein expression level, the number of the cells expressing the protein, and the occupied area of the cells expressing the protein within the tissue, using a "quantitative" technique, not a "qualitative" technique.

The "qualitative" technique refers to a technique in which the expression level of a protein, the number of cells expressing the protein, and the like, or index values closely related thereto, are not directly used, although correlated therewith; but instead, the numbers or the index values within a predetermined range are summarized and represented as one score, and about several, for example, from 2 to 5 levels of such scores are used for evaluation, based typically on the subjective and empirical judgement of the observer. For example, the IHC method using DAB staining and intended for detecting HER2 protein expressed on the cell membrane of breast cancer cells and the like, which method carries out the evaluation based on the stainability of the cell membrane of the cancer cells and the staining intensity (staining pattern) thereof according to the following four stage scores ("Guidelines for HER2 Testing, Third Edition" by Trastuzumab Pathology Committee, September, 2009), corresponds to the "qualitative" technique: 3+ (in cases where the ratio of cancer cells with an intense and complete positive staining of the cell membrane is > 30%: positive); 2+ (in cases where the ratio of cancer cells with a weak to moderate degree of complete positive staining of the cell membrane is ≥10%, or the ratio of cancer cells with an intense and complete positive staining of the cell membrane is ≥ 10% and ≤ 30%: equivocal); 1+ (in cases where the ratio of cancer cells with a barely recognizable, faint staining of the cell membrane is ≥ 10%, and the cancer cells are partially stained only at the cell membrane: negative); and 0 (in cases where no positive stain is observed in the cell membrane, or the ratio of cancer cells with a positive staining of the cell membrane is > 10% (positive staining localized only to the cell membrane is excluded from the evaluation): negative). Further, the technique disclosed in Non-patent Document 1 (page 20527, Figure 3) in which the expression level of a protein is evaluated according to four stage scores, based on a stained image obtained by the IHC method, also corresponds to the "qualitative" technique.

On the other hand, the "quantitative" technique refers to a technique in which the expression level of a protein and the number of cells expressing the protein, or index values closely related thereto, are directly used, and typically refers to a method based on objective measured results obtained using an apparatus. Representatively, a technique is used in which a target protein is labeled and quantified, using fluorescent nanoparticles, namely, particles having a nanosized diameter, for example, quantum dots (those which are not integrated), or particles obtained by integrating phosphors such as fluorescent dyes or quantum dots, using a resin, etc., as a matrix (Phosphor Integrated Dots: PIDs). In particular, a quantification method carried out using phosphor integrated dots (sometimes referred to as "PID method" in the present specification), which is used also in the Examples in the present specification, is particularly suitable as the "quantitative" technique to be used in the present invention. However, the "quantitative" technique which can be used in the present invention is not limited to the technique using phosphor integrated dots, such as the PID method, and other techniques having the same level of accuracy as that may also be used.

Basic embodiments of the PID method are known from the disclosures of WO 2012/029752 (Patent Document 1) and WO 2013/035703 (Patent Document 2), or other patent documents or non-patent documents. The PID method can be carried out also in the present invention, in an embodiment in accordance with the case of performing a pathology diagnosis using a sample slide, for example.

The "histogram" is prepared, as carried out in the Examples in the present specification, for example, by dividing the expression level per cell of the target protein into sections every predetermined number of particles (for example, the number of particles per cell of from one to 300 is divided every 20 particles, into 16 sections, including the section of 0), and plotting the number of cells (frequency) corresponding to each section. However, since the histogram is originally a graph obtained by measuring the expression level (the number of bright spots or the number of particles) of the protein and the number of cells expressing the protein, and directly using these values, the histogram is categorized as information obtained by a "quantitative" technique, not a "qualitative" technique.

In an example of a preferred embodiment, and in cases where the lesion site is a transplanted tumor site, namely, when the experimental animal is a tumor-bearing animal, the non-clinical test method according to the present invention further includes, in addition to the "post-transplant profile identification step" in which a specimen collected from the experimental animal is used: a profile identification step in which a specimen collected from a patient or cultured cells is used, namely, the step of identifying, using a specimen collected from a patient or cultured cells, the profile of tumor cells or tumor tissue before being transplanted into the experimental animal by the quantitative technique (sometimes referred to as a "pre-transplant profile identification step" in the present specification); and the step of comparing the profile identified by the pre-transplant profile identification step with the profile identified by the "post-transplant profile identification step" (sometimes referred to as a "profile comparison step" in the present specification). Such an embodiment allows for evaluating the extent to which the characteristics of the tumor cells or tumor tissue before being transplanted into the experimental animal are inherited and retained in the tumor site after being transplanted into the experimental animal, thereby enabling to more accurately carry out the quality control of experimental animals, the analysis of the drug efficacy, and the like.

The expression "tumor cells or tumor tissue before being transplanted" encompasses both the tumor tissue or tumor cells collected from a patient, and cultured cells derived from the tumor cells collected from the patient. It is preferred that the tumor cells or tumor tissue collected from a patient be used as the tumor cells or tumor tissue before being transplanted into the experimental animal, since it allows for eliminating the influence caused by the degeneration of cells during the culture. In other words, the non-clinical test method according to the present invention is preferably a method using an experimental animal transplanted with tumor tissue or tumor cells collected from a patient.

Further, the non-clinical test method according to the present invention may include the step of comparing the profiles of the transplanted tumor sites between experimental animals of the same or different generations. In an example of a preferred embodiment, the non-clinical test method according to the present invention further includes the steps of: identifying, using each of a specimen collected from an experimental animal of the 0th generation or the first generation and a specimen collected from an experimental animal of the second generation and beyond, the profile of the transplanted tumor site of each experimental animal by the quantitative technique; and comparing the profile of the tumor site of the experimental animal of the 0th generation or the first generation, with the profile of the tumor site of the experimental animal of the second generation and beyond, each of which profiles has been identified by the step just described above. Such an embodiment is useful, since it allows for evaluating the extent to which the characteristics of the transplanted tumor site are retained through passage in experimental animals, thereby enabling to more accurately carry out the quality control of the experimental animals, the analysis of the drug efficacy, and the like.

In a preferred embodiment, the non-clinical test method according to the present invention includes the step of collecting a specimen from the transplanted lesion site, such as a tumor site, of the experimental animal for a plurality of times, to identify the profile over time. For example, in cases where the experimental animal is a tumor-bearing model animal, a specimen is collected for a plurality of times, at time points immediately after the transplantation, after a certain period of time from the first specimen collection, and further, after a certain period of time from the second specimen collection, and the profile of each specimen can be to identified. By comparing the profiles of these specimens, it becomes possible to determine whether or not changes occur over time across the profiles of the same experimental animal, and thus, to more accurately evaluate, for example, the drug efficacy or the toxicity of a drug or a candidate substance thereof.

In order to collect a specimen from the experimental animal for a plurality of times, as described above, it becomes necessary to keep the experimental animal alive after collecting a specimen once; in other words, it is necessary to collect a specimen without sacrificing the experimental animal. The PID method, for example, has a high accuracy in quantifying a target protein and requires only a trace amount of specimen, and thus it is possible to collect a specimen from the tumor site without sacrificing the experimental animal. Further, when collecting a specimen from the tumor site, it is preferred to carry out a treatment for reducing haemorrhage from the collection site to a minimum level, such as, for example, freezing or heating a needle (biopsy needle) to be used for collecting the specimen so as to freeze or burn the tumor site, because it allows for reducing the damage to the experimental animal, and increasing the likelihood that the experimental animal survives after the collection of the specimen.

### EXAMPLES

### [Preparation Example 1] Preparation of Biotin-modified Anti-rabbit IgG Antibody

A quantity of 50 µg of an anti-rabbit IgG antibody as a secondary antibody was dissolved in a 50 mM Tris solution. To the resulting solution, a DTT (dithiothreitol) solution was added to a final concentration of 3 mM, followed by mixing, and the resultant was allowed to react at 37°C for 30 minutes. Subsequently, the reaction solution was allowed to pass through a desalting column "Zeba Desalt Spin Column" (Cat. #: 89882; manufactured by Thermo Fisher Scientific Inc.), to purify the secondary antibody which had been reduced with DTT. A quantity of 200 µL of the total amount of the purified antibody was dissolved in a 50 mM Tris solution, to prepare an antibody solution. Meanwhile, a linker reagent "Maleimide-PEG2-Biotin" (product number: 21901; manufactured by Thermo Fisher Scientific Inc.) was adjusted to a concentration of 0.4 mM with DMSO. A quantity of 8.5 µL of the thus prepared linker reagent solution was added to the antibody solution, followed by mixing. The resultant was allowed to react at 37°C for 30 minutes to bind biotin to the anti-rabbit IgG antibody via a PEG chain. The resulting reaction solution was purified by filtration through a desalting column. The absorbance of the desalted reaction solution was measured at a wavelength of 300 nm using a spectrophotometer ("F-7000" manufactured by Hitachi Ltd.) to calculate the concentration of the protein (biotin-modified secondary antibody) in the reaction solution. Using a 50 mM Tris solution, the concentration of the biotin-modified secondary antibody was adjusted to 250 µg/mL, and the thus prepared solution was used as a solution of the biotin-modified secondary antibody.

### [Preparation Example 2] Preparation of Texas Red Integrated Melamine Resin Particles

A quantity of 2.5 mg of Texas Red dye molecules "Sulforhodamine 101" (manufactured by Sigma-Aldrich Co. LLC.) was dissolved in 22.5 mL of pure water, and the resulting solution was then stirred by a hot stirrer for 20 minutes, while maintaining the temperature of the solution at 70°C. To the stirred solution, 1.5 g of a melamine resin "NIKALAK MX-035" (manufactured by Nippon Carbide Industries Co., Inc.) was added, and the mixture was further heated and stirred for five minutes under the same conditions. To the stirred solution, 100 µL of formic acid was added, and the mixture was stirred for 20 minutes while maintaining the temperature of the solution at 60°C, and the resulting solution was allowed to cool to room temperature. The cooled solution was dispensed into a plurality of centrifugation tubes, and then centrifuged at 12,000 rpm for 20 minutes to precipitate Texas Red integrated melamine resin particles contained in the solution as a mixture. Each supernatant was removed, and the precipitated particles were washed with Ethanol and water. An SEM observation was carried out for 1,000 resulting particles to measure the average particle size, and the average particle size of the particles was determined to be 152 nm. The thus prepared Texas Red integrated melamine resin particles were surface modified with streptavidin according to the following procedure, and the resulting streptavidin-modified Texas Red integrated melamine resin particles were used as phosphor integrated dots (PIDs) in Examples 1 and 3.

### [Preparation Example 3] Preparation of Streptavidin-modified Texas Red Integrated Melamine Resin Particles

A quantity of 0.1 mg of the particles obtained in Preparation Example 2 was dispersed in 1.5 mL of EtOH, followed by adding 2 µL of aminopropyltrimethoxysilane "LS-3150" (manufactured by Shin-Etsu Chemical Co., Ltd.) thereto, and the resultant was allowed to react for 8 hours to carry out a surface amination treatment.

Subsequently, PBS (phosphate buffered physiological saline) containing 2 mM EDTA (ethylenediaminetetraacetic acid) was used to prepare a solution of the particles which had been subjected to the surface amination treatment, having a concentration of 3 nM, and the resulting solution was mixed with SM (PEG) 12 (succinimidyl-[(N-maleimidopropionamido)-dodecaethyleneglycol] ester; manufactured by Thermo Fisher Scientific Inc.) to a final concentration of 10 mM, followed by a reaction for one hour. The thus obtained mixed liquid was centrifuged at 10,000 G for 20 minutes, and the supernatant was removed. PBS containing 2 mM EDTA was then added thereto to disperse the resulting precipitates, followed by another centrifugation. Washing by the same procedure was carried out three times, to obtain Texas Red integrated melamine resin particles with terminal maleimide groups.

Meanwhile, streptavidin (manufactured by Wako Pure Chemical Corporation) was subjected to a thiol group-addition treatment using N-succinimidyl S-acetylthioacetate (SATA), and the resultant was filtered through a gel filtration column, to obtain a solution of streptavidin capable of binding to Texas Red integrated melamine resin particles.

The above described Texas Red integrated melamine resin particles and streptavidin were mixed in PBS containing 2 mM EDTA, and the resultant was allowed to react at room temperature for one hour. Thereafter, 10 mM mercaptoethanol was added to terminate the reaction. After concentrating the resulting solution with a centrifugal filter, unreacted streptavidin and the like were removed using a gel filtration column for purification, to prepare streptavidin-modified Texas Red integrated melamine resin particles.

### [Preparation Example 4] Preparation of Melamine Resin Particles Encapsulating CdSe/ZnS Semiconductor Nanoparticles (Quantum Dots) Having Carboxylate (Carboxylate Group) as Surface Modification Group

To 7.5 g of tri-n-octylphosphine oxide, 2.9 g of stearic acid, 620 mg of n-tetradecylphosphonic acid, and 250 mg of cadmium oxide were added under a flow of argon, followed by heating to 370°C and mixing. After allowing the resulting mixture to cool to 270°C, a solution obtained by dissolving 200 mg of selenium in 2.5 mL of tributylphosphine was added thereto, and the resultant was dried under reduced pressure to obtain cadmium selenide (CdSe)-core semiconductor nanoparticles coated with tri-n-octylphosphine oxide.

To the resulting CdSe-core semiconductor nanoparticles, 15 g of tri-n-octylphosphine oxide was added, followed by heating. Then, while maintaining the temperature at 270°C, a solution obtained by dissolving 1.1 g of zinc diethyl dithiocarbamate in 10 mL of trioctylphosphine was added to the resultant, thereby obtaining a dispersion containing CdSe/ZnS semiconductor nanoparticles.

The resulting CdSe/ZnS semiconductor nanoparticles were dispersed in decane to a concentration of 5% by mass. To10 µL of the thus obtained dispersion, 0.5 mL of sodium propionate was added, followed by stirring at room temperature, to carry out surface modification. After adding 2.5 mL of pure water to the reaction mixture, the resulting solution was stirred by a hot stirrer for 20 minutes, while maintaining the temperature of the solution at 70°C. To the stirred solution, 1.5 g of a melamine resin "NIKALAK MX-035" (manufactured by Nippon Carbide Industries Co., Inc.) was added, and the mixture was further heated and stirred for five minutes under the same conditions.

To the stirred solution, 100 µL of formic acid was added, and the mixture was stirred for 20 minutes while maintaining the temperature of the solution at 60°C, and the resulting solution was allowed to cool to room temperature. The cooled solution was dispensed into a plurality of centrifugation tubes, and then centrifuged at 12,000 rpm for 20 minutes to precipitate melamine resin particles contained in the solution as a mixture, followed by removing each supernatant. Thereafter, the precipitated particles were washed with Ethanol and water. Thus, nanoparticles (quantum dot integrated melamine resin particles) having an average particle size of 150 nm were prepared. The thus prepared quantum dot integrated melamine resin particles were surface modified with streptavidin according to the procedure described in the following Preparation Example 5, and the resulting streptavidin-modified quantum dot integrated melamine resin particles were used as phosphor integrated dots (PIDs) in Example 2.

### [Preparation Example 5] Preparation of Streptavidin-modified Quantum Dot Integrated Melamine Resin Particles

A quantity of 0.1 mg of the particles obtained in Preparation Example 4 were dispersed in 1.5 mL of EtOH, followed by adding 2 µL of aminopropyltrimethoxysilane "LS-3150" (manufactured by Shin-Etsu Chemical Co., Ltd.) thereto, and the resultant was allowed to react for 8 hours to carry out a surface amination treatment.

Subsequently, PBS (phosphate buffered physiological saline) containing 2 mM EDTA (ethylenediaminetetraacetic acid) was used to prepare a solution of the particles which had been subjected to the surface amination treatment, having a concentration of 3 nM, and the resulting solution was mixed with SM (PEG) 12 (succinimidyl-[(N-maleimidopropionamido)-dodecaethyleneglycol] ester; manufactured by Thermo Fisher Scientific Inc.) to a final concentration of 10 mM, followed by a reaction for one hour. The thus obtained mixed liquid was centrifuged at 10,000 G for 20 minutes, and the supernatant was removed. PBS containing 2 mM EDTA was then added thereto to disperse the resulting precipitates, followed by another centrifugation. Washing by the same procedure was carried out three times, to obtain quantum dot integrated melamine resin particles with terminal maleimide groups.

Meanwhile, streptavidin (manufactured by Wako Pure Chemical Corporation) was subjected to a thiol group-addition treatment using N-succinimidyl S-acetylthioacetate (SATA), and the resultant was filtered through a gel filtration column, to obtain a solution of streptavidin capable of binding to fluorescent substance integrated melamine particles.

The above described quantum dot integrated melamine resin particles and streptavidin were mixed in PBS containing 2 mM EDTA, and the mixture was allowed to react at room temperature for one hour. Thereafter, 10 mM mercaptoethanol was added to terminate the reaction. After concentrating the resulting solution with a centrifugal filter, unreacted streptavidin and the like were removed using a gel filtration column for purification, to prepare streptavidin-modified quantum dot integrated melamine resin particles.

### [Example 1]

(1) PDX mice each transplanted with the breast cancer tissue (volume: about 200 mm³) of one of six patients (A to F) were purchased, five mice for each of the patients. To each of these PDX mice, 100 mg/kg of lapatinib (Trade name: Tykerb) was administered intravenously twice a day, for a total of 42 times. The measurement of the tumor volume was carried out before the administration and 21 days after the first administration. At the same time, a tissue section was collected from each mouse using a frozen needle, at each time point. The collected tissue sections were subjected to a formalin fixation treatment and a paraffin-embedding treatment, and then sliced to prepare sample slides, according to conventional methods. The thus prepared sample slides were used for the quantification of the expression level of HER2 as the target protein.
(2) The immunostaining of the sample slides with the phosphor integrated dots and the analysis of the expression level of HER2 protein (number of particles) were carried out by the following procedures.

### (2-1) Sample Preparation Step

Specimens were collected from the tumor sites of the purchased PDX mice according to a conventional method, and the specimens were then formalin fixed, paraffin-embedded and sliced, to prepare sample slides for the respective mice. Each of the thus prepared sample slides (n = 5 for each of the patients) was stained with Ventana benchmark ULTRA using a Ventana I-VIEW Pathway HER2 (4B5) kit, to determine the HER2 score by the DAB method, and the tumor cell area and the stromal cell area in each sample slide were morphologically identified. The HER2 score was determined in accordance with a commonly used standard (see, for example, "Guidelines for HER2 Testing, Third Edition" by Trastuzumab Pathology Committee, September, 2009). In Example 1, one specimen (A) had an area with a negative score; one specimen (B) had an area with a score of 1+; one specimen (C) had an area with a score of 2+; and three specimens (D, E and F) had an area with a score of 3+.

After subjecting the sample slides to deparaffinization treatment, the slides were subjected to a displacement washing with water. An antigen activation treatment was carried out by subjecting the washed sample slides to an autoclave treatment in a 10 mM citric acid buffer solution (pH 6.0) at 121°C for 15 minutes. The tissue array slides after being subjected to the antigen activation treatment were washed with PBS, and the blocking treatment of the washed tissue array slides was carried out for one hour, using PBS containing 1% BSA.

### (2-2) Immunostaining Step

### (2-2-1) Primary Reaction Treatment of Immunostaining

Using PBS containing 1W/W% BSA, a primary reaction treatment liquid containing an anti-HER2 rabbit monoclonal antibody "4B5" (manufactured by Ventana Medical Systems) at a concentration of 0.05 nM was prepared, to be used in a primary reaction treatment for the first immunostaining of the target protein, HER2. The sample slides prepared in the step (1) were immersed in the thus prepared primary reaction treatment liquid, and allowed to react at 4°C overnight.

### (2-2-2) Secondary Reaction Treatment of Immunostaining

The solution of the biotin-modified anti-rabbit IgG antibody prepared in Preparation Example 1 was further diluted to a concentration of 6 µg/mL, using PBS containing 1W/W% BSA, to prepare a secondary reaction treatment liquid. The sample slides after being subjected to the primary reaction treatment were washed with PBS, and then immersed in the thus prepared secondary reaction treatment liquid, and allowed to react at room temperature for 30 minutes.

### (2-2-3) Fluorescent Labeling Treatment of Immunostaining

The streptavidin-modified Texas Red integrated melamine resin particles prepared in Preparation Example 3 was diluted to a concentration of 0.02 nM, using a diluent for fluorescent nanoparticles containing casein and BSA, to prepare a fluorescent labeling reaction treatment liquid. The sample slides after being subjected to the secondary reaction treatment were immersed in the thus prepared fluorescent labeling treatment liquid, and allowed to react at room temperature for three hours, in a neutral pH environment (pH of from 6.9 to 7.4).

### (2-2-4) Staining Treatment for Morphological Observation

The sample slides after being subjected to the fluorescent labeling treatment were stained with a Mayer's hematoxylin liquid for five minutes to carry out hematoxylin staining, followed by washing with running water at 45°C for three minutes.

### (2-3) Sample Post-treatment Step

The sample slides which had been immunostained were subjected to an immobilization and dehydration treatment, in which an operation of immersing the slides in pure Ethanol for five minutes was repeated four times. Subsequently, the sample slides were subjected to a clearing treatment, in which an operation of immersing the slides in xylene for five minutes was repeated four times. Finally, a sealing treatment was carried out in which a sealant "Entellan New" (manufactured by Merck KGaA) was applied on the samples and coverslips were placed thereover, and the resultants were used as samples for use in observation.

### (2-4) Evaluation Step

### (2-4-1) Observation and Image Capture Step

A fluorescence microscope, "BX-53" (manufactured by Olympus Corporation), was used for the irradiation of an excitation light and the observation of fluorescence emission in this step; and a digital camera for a microscope, "DP73" (manufactured by Olympus Corporation), attached to the fluorescence microscope was used for capturing immunostained images (400-fold).

First, an excitation light corresponding to the Texas Red dye used for the fluorescent labeling of the target protein HER2 was irradiated to each sample to allow fluorescence emission to occur, and an immunostained image of the sample in that state was captured. At this time, the wavelength of the excitation light was set within the range of from 575 to 600 nm, using an optical filter for excitation light equipped in the fluorescence microscope, and the wavelength of the fluorescence to be observed was adjusted within the range of from 612 to 692 nm, using an optical filter for fluorescence. The intensity of the excitation light during the observation and image capture by the fluorescence microscope was adjusted such that the irradiation energy in the vicinity of the center of the visual field was 900 W/cm². The exposure time during the image capture was adjusted within a range such that the brightness of the image to be captured was not saturated, such as, for example, to 4000 µ seconds.

Next, the observation and the image capture in the bright field of the fluorescence microscope were carried out, to obtain hematoxylin-stained images for morphological observation of the cells.

The capturing of the immunostained images and the stained images for morphological observation as described above were carried out in the same visual field, and then the same operation was repeated, changing the visual field each time, to obtain images captured in five different visual fields per one sample slide.

### (2-4-2) Image Processing and Measurement Step

Image processing software "ImageJ" (open source) was used for the image processing to be carried out in this step.

For each of the samples, the image processing using the stained image for morphological observation was carried out to identify the shape (the position of the cell membrane) of the cells, and the stained image was overlaid with the immunostained image, to extract bright spots indicating the Texas Red integrated melamine resin particles labeling the HER2 protein expressed on the cell membrane. Further, since HER2 is not expressed in the stromal cell area, the bright spots present within the stromal cells were processed as non-specific signals, namely, the noise. The number of bright spots on the cell membrane having a brightness equal to or greater than a predetermined value was counted, and the brightness of the bright spots was divided by the brightness per one particle of the above described phosphor integrated dots (PIDs) to be converted into the number of particles, and the thus obtained value was taken as the expression level of HER2 in the cell. Subsequently, the expression level of HER2 (number of particles) was measured for 1,000 cells per one sample slide (five visual fields), and the mean value thereof was calculated to be taken as the "PID score" of the sample slide. Further, the mean value of the PID scores of five pieces of sample slides corresponding to each of the patients (A to F) was calculated.

(3) The results are as shown in Table 2. As a result, the difference in the expression level of the protein which was not detectable by the DAB score (qualitative evaluation value) was revealed by the PID score (quantitative evaluation value) measured using the phosphor integrated dots. Further, it has been confirmed, in the area with a large expression level of HER2 protein, that a higher reduction in the tumor size as a result of lapatinib administration as well as an increased drug efficacy (effect of reducing the tumor size) of lapatinib tend to be observed in a PDX mouse having a higher PID score, among the PDX mice having the same DAB score of 3+. Such an analysis is useful, for example, in that it becomes possible to stratify and identify PDX mice for which lapatinib is more effective.

**[Table 2]**

| | Before first Lapatinib administration | | 21 days after first Lapatinib administration |
|---|---|---|---|
| Sample | PID score | DAB score (HER2) | Tumor size (size at the start of administration is taken as 100%) |
| A | 10 | - | 180% |
| B | 30 | 1+ | 120% |
| C | 100 | 2+ | 80% |
| D | 200 | 3+ | 70% |
| E | 400 | 3+ | 60% |
| F | 800 | 3+ | 10% |

### [Example 2]

PDX mice each transplanted with the breast cancer tissue (volume: about 200 mm³) of either of two patients determined as 3+ in the DAB staining were prepared, five mice for each of the patients (Samples X and Y). To each of these PDX mice, 15 mg/kg of trastuzumab (trade name: Herceptin) was administered intravenously once a day, for a total of three times, and the tumor volume was measured before the first administration, 3 days after the first administration, 1 week and 2 weeks after the first administration (Samples X); or 1 week, 2 weeks and 3 weeks after the first administration (Samples Y). At the same time, a tissue section was collected from each mouse using a frozen needle, at each time point. The collected tissue sections were subjected to a formalin fixation treatment and a paraffin-embedding treatment, and then sliced to prepare sample slides, according to conventional methods. The thus prepared sample slides were used for the quantification of the expression level of HER2 as the target protein. The immunostaining of the sample slides with the phosphor integrated dots and the analysis of the expression level of HER2 protein (number of particles) were carried out in the same manner as in Example 1, except that the streptavidin-modified quantum dot integrated melamine resin particles prepared in Preparation Example 5 were used instead of the streptavidin-modified Texas Red integrated melamine resin nanoparticles prepared in Preparation Example 3, in the fluorescent labeling treatment of the immunostaining.

The results are as shown in Table 3 and Table 4. As were the results of Example 1, the difference in the expression level of the protein which was not detectable by the DAB score was revealed by the PID score. Further, the drug efficacy (tumor reducing effect) and the behavior of the protein expression level were different between the two types of mice, and there is a possibility that these results may contribute to the analysis of the mechanism responsible therefor.

**[Table 3]**

| Sample X | PID score | DAB score (HER2) | Tumor size (size at the start of administration is taken as 100%) |
|---|---|---|---|
| Before administration | 200 | 3+ | 100% |
| After 3 days | 200 | 3+ | 90% |
| After 1 week | 180 | 3+ | 80% |
| After 2 weeks | 140 | 3+ | 50% |

**[Table 4]**

| Sample Y | PID score | DAB score (HER2) | Tumor size (size at the start of administration is taken as 100%) |
|---|---|---|---|
| Before administration | 200 | 3+ | 100% |
| After 3 days | 160 | 3+ | 80% |
| After 1 week | 80 | 3+ | 90% |

### [Example 3]

PDX mice were each transplanted with the breast cancer tissue (volume of about 200 mm³) of a patient determined as 3+ in the DAB staining, and then passaged to prepare five first generation PDX mice (Samples PI). Further, two types of the second generation PDX mice (P2-1 and P2-2) were prepared, five mice for each type. Subsequently, using tumor tissue collected from each of the P2-1 and P2-2 mice, two types of the third generation PDX mice (Samples P3-1 and P3-2) were prepared, five mice for each type.

To each of the PDX mice, 15 mg/kg of trastuzumab (trade name: Herceptin) was administered intravenously once a week, for a total of five times, and the tumor volume was measured before the first administration, and one month after the first administration. At the same time, a tissue section was collected from each mouse using a frozen needle, at each time point. The collected tissue sections were subjected to a formalin fixation treatment and a paraffin-embedding treatment, and then sliced to prepare sample slides, according to conventional methods. The thus prepared sample slides were used for the quantification of the expression level of HER2 as the target protein. The immunostaining of the sample slides with the phosphor integrated dots and the analysis of the expression level of HER2 protein (number of particles) were carried out in the same manner as in Example 1. In addition, a frequency curve (histogram) was prepared for each of the Samples, using the mean value of the number of the particles in each Sample.

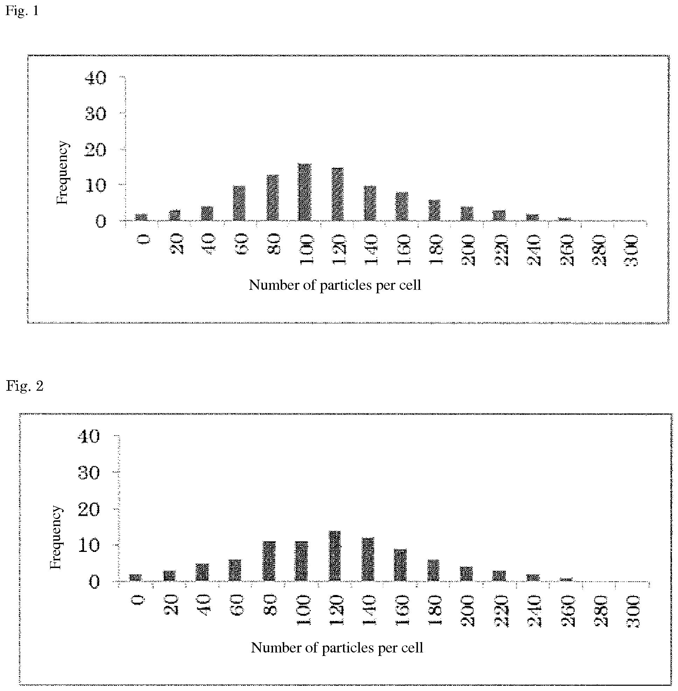

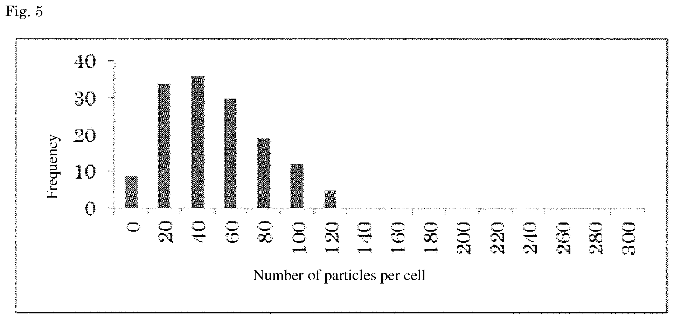

The results are as shown in Table 5 and FIGs. 1 to 5. A high drug efficacy (tumor-reducing effect) was observed in the PDX mice P2-1 (FIG. 2) and P3-1 (FIG. 4), whose histograms coincide with that of the breast cancer tissue of the patient (FIG. 1). Note that a histogram was determined to coincide with that of the tissue of the patient, when all of the following requirements were satisfied: (i) mean value: within 15%; (ii) number of peaks: the same number; and (iii) difference in CV value: within 5%. By comparing the histograms showing the protein expression levels per cell, based on the results as described above, it is possible to select mice which are capable of retaining and passing on the characteristics of tumor to the next generations.

**[Table 5]**

| Sample | Histogram | Tumor size after drug administration (size at the start of administration is taken as 100%) |
|---|---|---|
| P1 (First generation mice) | FIG. 1 | 50% |
| P2-1 (Second generation mice 1) | FIG.2 | 50% |
| P2-2 (Second generation mice 22) | FIG.3 | 110% |
| P3-1 (Third generation mice produced from P2-1) | FIG.4 | 60% |
| P3-2 (Third generation mice produced from P2-2) | FIG.5 | 120% |

### [Example 4]

CD140a is a membrane protein which is expressed on the surface of cells such as fibroblasts, megakaryocytes, monocytes, erythrocytes, myeloid progenitor cells and endothelial cells, and is used as a stromal cell marker. The expression levels of CD140a in the tumor tissues of PDX mice were measured to select mice, and the evaluation of the drug efficacy of an anticancer drug in the thus selected mice were carried out.

### < Selection of Mice>

PDX mice each transplanted with the breast cancer tissue (volume: about 200 mm³) of one of six patients (A to F), which were the same as those used in Example 1, were purchased, five mice for each of the patients. These PDX mice were reared, and the size of the tumor in each mouse was measured every 3 or 4 days. When the tumor volume reached 500 mm³, tumor tissue was collected from each mouse using a scalpel.

### < Measurement of Expression Level of CD140a >

Using the sections of the tumor tissues of the PDX mice produced as described above, each having a size of about 3 mm square, sample slides were prepared, and the activation and blocking treatments of the slides were carried out, in the same manner as in Example 1.

The thus prepared sample slides were subjected to a primary immune reaction in the same manner as in Example 1, except that the primary reaction treatment liquid was prepared using a biotin-modified anti-mouse CD140a rat monoclonal antibody (clone: APA5, manufactured by Biolegend, Inc.), instead of the anti-HER2 rabbit monoclonal antibody "4B5" (manufactured by Ventana Medical Systems). After washing the sample slides with PBS, the fluorescent labeling treatment was carried out using the streptavidin-modified Texas Red-integrated melamine resin body-integrated particles (PIDs) prepared in Preparation Example 3. Further, the staining treatment for morphological observation and the sample post-treatment were carried out in the same manner as in Example 1 (2-2-3), to obtain sample slides to be used for observation (CD140a/ PID stained sample slides).

The observation and image capture of the immunostained images and the stained images for morphological observation were carried out in the same manner as in Example 1, to measure the PID score or the DAB score of CD140a. From each group consisting of five mice transplanted with the tumor tissue of one of the respective patients, three mice with a low PID score or DAB score of the CD140a, namely, mice with a low ratio of mouse-derived stromal cells were selected to be used in the following drug efficacy test. The selection of mice using any marker, as described above, allows for an experiment with a higher reproducibility. Further, by eliminating mice which had not been selected in the drug efficacy test, it is possible to reduce the number of experimental animals unnecessarily used, which is preferred also from the viewpoint of animal protection.

### < Evaluation of Drug Efficacy of Anticancer Drug >

To each of the thus selected three PDX mice, in each group, 100 mg/kg of lapatinib (trade name: Tykerb) was administered intravenously twice a day, for a total of 42 times, in the same manner as in Example 1, and the tumor volume was measured before the first administration, and 21 days after the first administration. At the same time, a tissue section was collected from each mouse using a frozen needle, at each time point. The collected tissue sections were used for the quantification of the expression level of HER2 protein as the target protein, in the same manner as in Example 1.

As were the results of Example 1, it has been confirmed that a higher reduction in the tumor size as a result of lapatinib administration as well as an increased drug efficacy (effect of reducing the tumor size) of lapatinib tend to be observed in a PDX mouse having a higher PID score of HER2 protein.

### [Example 5] Hemato-lymphoid Humanized Model Mice

Hemato-lymphoid humanized mice, produced by transplanting human hematopoietic stem cells into mice with acquired immune deficiency, were transplanted with tumor tissue, to prepare hemato-lymphoid humanized model mice, which are one type of patient-derived tumor-transplanted mice. Tumor tissues collected from six lung cancer patients were purchased from SofiaBio, and the thus produced hemato-lymphoid humanized mice were each subcutaneously transplanted with the tumor tissue, having a size of 2 mm square, of one of the patients (five mice transplanted with the tumor tissue of one patient were produced for each of the patients). To each of the model mice whose tumor tissue had grown to the size of about 300 mm³ one month after the transplantation, 100 mg/kg of an anti-human PD-1 monoclonal antibody preparation, nivolumab (trade name: Opdivo) was administered intravenously twice a day, for a total of 42 times. In the same manner as in Example 1, the tumor volume was measured before the first administration and 21 days after the first administration. At the same time, a tissue section was collected from each of the mice, and sample slides were prepared in the same manner as in Example 1. The staining and observation of the sample slides were carried out in the same manner as in Example 1, except that an anti-PD-Ll rabbit monoclonal antibody (clone "SP142"; manufactured by Spring Bioscience (SBS) Corporation) was used instead of the anti-HER2 rabbit monoclonal antibody "4B5" (manufactured by Ventana Medical Systems), in the primary reaction treatment of the immunostaining.

As were the results of Example 1, it has been confirmed that a higher reduction in the tumor size as a result of lapatinib administration as well as an increased drug efficacy (effect of reducing the tumor size) of nivolumab tend to be observed in a PDX mouse having a higher PID score of PD-L1 protein.

### [Example 6] Orthotopic Transplantation of Tumor in Immune-PDX Model Mice

Tumor tissues collected from three lung cancer patients with advanced lymph node metastasis were purchased from SofiaBio. The tumor tissue, having a size of 2 mm square, of one of the patients was transplanted into the subcutaneous tissue or lung tissue of each of the mice with acquired immune deficiency which had been transplanted with tumor-infiltrating lymphocytes, to produce Immune-PDX model mice (five mice transplanted with the tumor tissue of one patient were produced for each of the patients). One month later, the tissue of cancer which had grown to a size of about 300 mm³ and lymph node tissue were collected from each of the mice, and sample slides were prepared in the same manner as in Example 1.

The staining and observation of these sample slides were carried out in the same manner as in Example 1, except that an anti-MET rabbit monoclonal antibody (SP44; manufactured by Hoffmann-La Roche Inc.) was used instead of the anti-HER2 rabbit monoclonal antibody "4B5" (manufactured by Ventana Medical Systems), in the primary reaction treatment of the immunostaining.

As a result, the metastasis of the tumor in the lymph node tissue was observed, in the morphological observation of the tissue slides prepared with the lymph node tissues obtained from the mice which had been orthotopically transplanted with the tumor tissues. In contrast, no tumor metastasis in the lymph node tissue was observed in all the tissue slides prepared with the lymph node tissues obtained from the mice which had been subcutaneously transplanted with the tumor tissues. The above results indicate that the state of metastasis varies depending on the site to which the tumor is transplanted when producing tumor transplanted model mice.

Further, in the fluorescence observation of the tissue slides prepared with the tissues obtained from the orthotopically transplanted mice, bright spots were generally observed in a number equal to or greater than twice the number of those observed in the subcutaneously transplanted tumor tissues, revealing that the expression of the cancer progression marker, MET, differs between the orthotopically transplanted tumors and the subcutaneously transplanted tumors. This indicates that, in the subcutaneously transplanted mice, an accurate evaluation cannot be made whether the metastasis occurs according to the characteristics of the original tumors of the respective patients. In the orthotopically transplanted model mice, in contrast, it can be evaluated that the characteristics of the transplanted tumors are retained, and the metastasis occurs as the tumors grow. These results suggest that there is a possibility that the quality control of the tumor-bearing mice and the analysis of the results of the tests using these mice, as described above, can be carried out more accurately, by evaluating whether the transplanted tumor sites retain and inherit the characteristics of the tumors before being transplanted, as described above.

## Claims

1. A non-clinical test method comprising the step of identifying, using a specimen collected from an experimental animal, the profile of a lesion site of the experimental animal by a quantitative technique.

2. The non-clinical test method according to claim 1, wherein the lesion site is a transplanted tumor site.

3. The non-clinical test method according to claim 2, further comprising the steps of:
identifying, using a specimen collected from a patient or cultured cells, the profile of tumor cells or a tumor tissue before being transplanted into the experimental animal by the quantitative technique; and
comparing the profile identified by the step just described above with the profile of the transplanted tumor site of the experimental animal.

4. The non-clinical test method according to claim 3, wherein the tumor cells or the tumor tissue before being transplanted into the experimental animal are/is tumor cells or tumor tissue collected from the patient.

5. The non-clinical test method according to claim 2, the method comprising the steps of:
an identifying step, using each of a specimen collected from an experimental animal of the 0th generation or the first generation and a specimen collected from an experimental animal of the second generation and beyond, the profile of the transplanted tumor site of each experimental animal by the quantitative technique; and
a comparing step, the profile of the tumor site of the experimental animal of the 0th generation or the first generation, with the profile of the tumor site of the experimental animal of the second generation and beyond, each of which profiles has been identified by the identifying step.

6. The non-clinical test method according to any one of claims 2 to 5, the method comprising the step of determining the experimental animal from which the tumor site is collected for passage, or the location of the tumor site to be collected, based on the profile identified by the identifying step.

7. The non-clinical test method according to any one of claims 1 to 6, wherein the profile comprises information regarding an average expression level per cell of a target protein in the specimen.

8. The non-clinical test method according to any one of claims 1 to 7, wherein the profile comprises information regarding a expression level per unit area of the tissue, of the target protein in the specimen.

9. The non-clinical test method according to any one of claims 1 to 8, wherein the profile comprises information regarding a histogram showing the expression level per cell of the target protein and the number of cells corresponding thereto, in the specimen.

10. The non-clinical test method according to any one of claims 1 to 8, wherein the profile comprises information regarding a curve showing the expression level per cell of the target protein and the number of cells corresponding thereto, in the specimen.

11. The non-clinical test method according to any one of claims 1 to 10, wherein immunostaining using fluorescent nanoparticles is carried out as the quantitative technique for identifying the profile.

12. The non-clinical test method according to any one of claims 1 to 11, wherein the profile comprises information regarding the vascular occupancy in the specimen.

13. The non-clinical test method according to any one of claims 7 to 12, wherein the target protein is at least one selected from the group consisting of immune checkpoint proteins, cancer cell growth factors, cancer cell growth factor receptors, cell surface antigens, vascular growth factors, vascular growth factor receptors, cytokines and cytokine receptors.

14. The non-clinical test method according to any one of claims 1 to 13, the method comprising the step of collecting a specimen from the transplanted lesion site of the experimental animal for a plurality of times, to identify the profile.

15. The non-clinical test method according to any one of claims 1 to 14, wherein a frozen or heated needle is used when collecting the specimen.

16. The non-clinical test method according to any one of claims 7 to 15, wherein the target protein could be a marker targeted by a molecular target drug, and wherein the profile comprises the expression level and the expression distribution of the marker.

17. The non-clinical test method according to any one of claims 7 to 15, wherein the target protein is a phosphorylated protein, and wherein the profile comprises the expression level and the expression distribution of the phosphorylated protein.
